# EUROPEAN PATENT APPLICATION

(11) **EP 4 342 908 A1**
(43) Date of publication of application: **27.03.2024**
(21) Application number: 22823813.5
(22) Date of filing: 03.03.2022
(51) Int. Cl.: C07K 16/00, C12P 21/08

(54) **CHIMERIC IMMUNOGLOBULIN**

(30) Priority: 17.06.2021 CN 202110674660
(71) Applicant: Fapon Biotech Inc., Shenzhen, Guangdong 518000 (CN)
(72) Inventor: MENG, Yuan, Dongguan, Guangdong 523000 (CN); ZHONG, Dongmei, Dongguan, Guangdong 523000 (CN)
(74) Representative: V.O.
(86) International application number: PCT/CN2022/078975
(87) International publication number: WO 2022/262321

(57) **Abstract**

A chimeric immunoglobulin is provided. It is obtained by fusing a first polypeptide and a second polypeptide, wherein the first polypeptide includes an IgG variable region located at the N terminal; and the second polypeptide includes an IgA CH2-CH3 region or an IgM CH3-CH4 region located at the C terminal. The immunoglobulin has higher titer, and when coated in a solid phase, it still has higher titer than that of an IgG monomer with the same valence, so it can be widely applied in the detection field.

## Description

### Cross-Reference to Related Application

The present application claims priority of Chinese patent application No. 202110674660.8 filed on Jun 17, 2021 and entitled "CHIMERIC IMMUNOGLOBULIN", the entire content of which is incorporated herein by reference.

### Technical Field

The present application relates to the field of antibody engineering, and in particular to a chimeric immunoglobulin.

### Background

An immunoglobulin (Ig) is composed of four polypeptide chains, each polypeptide chain being connected by a different number of interchain disulfide bonds. The Ig can be of a "Y"-shaped structure, referred to as an Ig monomer, and is a basic unit of an antibody. A natural Ig molecule contains four heterologous polypeptide chains, of which two chains with larger molecular weights are called heavy chains (Hs) and two chains with smaller molecular weights are called light chains (Ls). The amino acid compositions of two heavy chains and two light chains in the same Ig molecule are exactly the same. The heavy chain has a molecular weight of 50,000-75,000 and consists of 450-550 amino acid residues. The antigenicity of the Ig is different because of a different amino acid composition and arrangement order in a heavy chain constant (CH) region. According to this, the Ig can be divided into five classes, i.e., IgM, IgD, IgG, IgA and IgE. In the five classes, IgG, IgD and IgE are all of monomer structures. The IgM is a basic antibody secreted by B cells. In a secretory morphology, the IgM is a pentamer form composed of five Y-shaped monomers and has extremely high affinity. The IgM has a very large molecular weight, so it is very effective in an antigen agglutination reaction. The IgM is a multivalent antibody. The IgA can exist in the form of a monomer or dimer, trimer or tetramer. A secretory IgA mostly exists in the form of a dimer and trimer, and it is also a multivalent antibody. Because a monomer antibody has 2 antigen binding sites, the IgA usually has 4 or 6 antigen binding sites, while the IgM has 10 or 12 antigen binding sites, both of which are multivalent antibodies.

The antibody is mainly synthesized by B lymphocytes. Each B lymphocyte has a genetic gene for synthesizing an antibody. When the body is stimulated by an antigen, many determinants on the antigen molecule activates various B cells having different genes respectively. The activated B cells divide and proliferate to form multiple clones and synthesize various antibodies. In 1975, British scientists, Kohler and Milstein, were cooperated to fuse mouse myeloma cells which had been adapted to in vitro culture with mouse splenocytes (B lymphocytes) immunized with sheep erythrocytes. It was found that the formed hybrid cells could not only proliferate indefinitely in vitro, but also secrete specific antibodies continuously, and pure cells obtainable through clonal culture could produce monoclonal antibodies, thereby inventing monoclonal antibody technology. The emergence of the monoclonal antibody technology is a major breakthrough in the field of immunology. By the mid-1980s, the monoclonal antibody technology had been perfected day by day, and began to be widely applied in many fields such as biomedical research, biotechnology, clinical diagnosis and treatment, and the like.

With the progress of science and technology, in vitro diagnosis has become an important helper of clinical medical diagnosis, playing the role of doctor's eyes. The monoclonal antibody, as one of the core raw materials of in vitro diagnostic reagents in clinical medicine, gives full play to its advantages. The monoclonal antibody has strong specificity, which greatly improves the specificity of an antigen-antibody reaction, reduces the possibility of cross-reaction with other substances, and makes the test results more reliable. The homogeneity and biological activity unicity of the monoclonal antibody make the result of the antigen-antibody reaction easy to control, which is beneficial to standardization and normalization.

An immunodiagnostic reagent is a subdivision field of in vitro diagnostic reagents. Immunodiagnosis is the application of immunological theories, techniques and methods to diagnose various diseases and determine an immune status. Immunodiagnostic methods include radioimmunoassay, enzyme-linked immunosorbent assay, chemiluminescence, fluorescence chromatography or colloidal gold chromatography, etc. The basic principles of these methods are described hereafter. An antigen or antibody is bound to a surface of a solid support while maintaining its immune activity. A suitable marker is selected to form a labeled antigen or antibody complex with the antigen or antibody by a chemical method. During determination, a specimen to be tested (for determining the antibody or antigen contained therein) and the labeled antigen or antibody complex react with the antigen or antibody on the surface of the solid support according to different steps. An antigen-antibody complex formed on the solid support is separated from other substances by washing, and the labeled complex finally bound on the solid support is in a certain proportion to the amount of the substance to be detected in the specimen. After adding, a substrate that reacts with the labeled complex is catalyzed to become a colored product. Because the amount of the colored product is directly related to the amount of the substance to be detected in the specimen, qualitative or quantitative analysis can be carried out according to the depth of a color reaction. However, currently the improvement of antibody titer used for immunodiagnosis still mostly depends on the improvement of an antibody immunization and screening process, and its effect is very random, which cannot meet the detection needs very well.

### SUM MARY

A first aspect of the present application relates to an immunoglobulin which is obtained by fusing a first polypeptide and a second polypeptide, wherein
the first polypeptide includes an IgG variable region located at the N terminal; and
the second polypeptide includes an IgA CH2-CH3 region or an IgM CH3-CH4 region located at the C terminal.

A second aspect of the present application relates to a multimer, which is obtained by polymerizing the immunoglobulin described above as a monomer.

A third aspect of the present application relates to an isolated nucleic acid encoding the immunoglobulin described above.

A fourth aspect of the present application relates to a vector containing the nucleic acid described above.

A fifth aspect of the present application relates to a host cell containing the nucleic acid described above or transformed with the vector described above.

A sixth aspect of the present application relates to a method for preparing an immunoglobulin, which includes expressing the host cell described above or subjecting the first polypeptide and the second polypeptide described above to fusion expression, and relates to an immunoglobulin prepared by the preparation method described above.

A seventh aspect of the present application relates to a method for preparing a multimer, which includes polymerizing an immunoglobulin prepared by the aforementioned method for preparing an immunoglobulin as a monomer, and relates to a multimer prepared by the preparation method described above.

A eighth aspect of the present application relates to a solid support, the surface of which is coated with the multimer described above.

A ninth aspect of the present application relates to a kit or test strip containing the multimer described above or the solid support described above.

A tenth aspect of the present application relates to a method for detecting an antigen, which includes contacting the antigen with a multimer described above or a solid support described above to form a conjugate, and detecting the conjugate;
wherein the antigen is an antigen capable of specifically binding with the IgG variable region.

An eleventh aspect of the present application relates to use of the multimer, solid support, kit or test strip in immunoassay.

The IgG and IgM/lgA chimeric immunoglobulins provided by the present application have higher titer, and when coated in a solid phase, they still have higher titer than that of an IgG monomer with the same valence, so they can be widely applied in the detection field.

Details of one or more implementations of the present application are set forth in the following drawings and description. Other features, objectives and advantages of the present application will be apparent from the description, drawings and claims.

### Brief Description of the Drawings

In order to illustrate the specific embodiments of the present application or the technical solution in the prior art more clearly, the following will briefly introduce the drawings needed to be used in the detailed description or the description of the prior art. Obviously, the drawings in the following description are some embodiments of the present application, and for those of ordinary skills in the art, other drawings can be obtained according to these drawings without creative labor.
Fig. 1 shows electrophoresis results of MA-9G7TB1 and MA-9G7TB2 IgG/IgM heterozygous recombinant antibodies in one embodiment of the present application;
Fig. 2 shows the electrophoresis result of a 3E50TB2 heterozygous recombinant antibody in one embodiment of the present application;
Fig. 3 shows the electrophoresis result of a 5F27TB2 heterozygous recombinant antibody in one embodiment of the present application; and
Fig. 4 shows the electrophoresis result of a 21C5TB2 heterozygous recombinant antibody in one embodiment of the present application.

### Detailed Description of the Embodiments

Reference will now be made in detail to implementations of the present application, one or more examples of which are described hereafter. Each example is provided as an explanation, rather than as a limitation of the present application. In fact, it is obvious to those skilled in the art that many modifications and changes can be made to the present application without departing from the scope or spirit of the present application. For example, features illustrated or described as part of one implementation can be used in another implementation to yield a further implementation.

Unless otherwise defined, all technical and scientific terms used herein have the same meanings as those commonly understood by those skilled in the art to which the present application pertains to. Herein, the terminology used in this specification of the present application is only for the purpose of describing specific examples, and is not intended to limit the present application. As used herein, the term "and/or" includes any and all combinations of one or more related listed items.

During the experiment, the inventors of the present application have unexpectedly found that a phenomenon of polymerization of normal IgG antibodies will occur with the change of storage conditions such as temperature and buffer conditions. The appearance of a multimeric antibody is directly embodied in the fact that the activity exhibited by the antibody is much higher than that of a monomer antibody during the application of a diagnostic reagent. However, naturally occurring IgG polymers are unstable. Combining the characteristics of IgA and IgM antibodies and the hydrophilic and hydrophobic characteristics of their constant regions as well as the objective of obtaining uniform and highly expressed multimeric antibodies, the inventors have found that the performance of the constructed IgG/lgA and IgG/IgM chimeric antibodies is much higher than that of IgG antibodies in the process of detecting with the diagnostic reagents, and the chimeric antibodies have very good advantages in homogeneity and expression amount.

Therefore, IgG/lgA and IgG/IgM chimeric antibodies are constructed and applied to a coated or labeled terminal of an in vitro diagnostic detection reagent. The IgG/lgA and IgG/IgM chimeric antibodies in the present application can replace the existing IgG antibodies, thereby obtaining a detection kit with better activity.

In a first aspect, an implementation of the present application provides an immunoglobulin which is obtained by fusing a first polypeptide and a second polypeptide, wherein
the first polypeptide includes an IgG variable region located at the N terminal; and
the second polypeptide includes an IgA CH2-CH3 region or an IgM CH3-CH4 region located at the C terminal.

In some implementations, the IgG in the first polypeptide further has a CH1 region.

In some implementations, the IgA or the IgM in the second polypeptide further has a CH1 region.

In some implementations, the first polypeptide further includes a hinge region of the IgG.

In some implementations, the second polypeptide further includes a hinge region of the IgA or the IgM.

In some implementations, the IgG in the first polypeptide further has a CH1-CH2 region.

In some implementations, the IgG in the first polypeptide further has a CH1-CH2-CH3 region.

In some implementations, the IgM in the second polypeptide further has a CH1-CH2 region.

In some implementations, the IgG is IgG1, IgG2, IgG3 or IgG4.

In some implementations, at least one of the first polypeptide and the second polypeptide includes a CH1 region.

In some implementations, at least one of the first polypeptide and the second polypeptide includes a hinge region.

The IgG, IgA and IgM can be independently selected from the same or different species sources, for example murine (mice, rats), rabbits, sheep, goats, horses, chickens, cows, dogs and humans.

In a second aspect, implementations of the present application provide a multimer, which is obtained by polymerizing the immunoglobulin described above.

In some implementations, the multimer is obtained by polymerizing 2, 3 or 4 immunoglobulin monomers having IgA CH2-CH3 regions.

In some implementations, the multimer is obtained by polymerizing 5 immunoglobulin monomers having IgM CH3-CH4 regions.

In some implementations, the multimer is further conjugated with a signal substance.

In a third aspect, implementations of the present application provide an isolated nucleic acid encoding the immunoglobulin described above.

In a fourth aspect, implementations of the present application further provide a vector containing the nucleic acid described above.

The term "vector" refers to a nucleic acid vehicle into which polynucleotides can be inserted. When the vector can express a protein encoded by the inserted polynucleotides, it is called an expression vector. The vector can be introduced into a host cell by transformation, transduction or transfection, so that a genetic material element carried by the vector can be expressed in the host cell. The vector is well known to those skilled in the art, including but not limited to: a plasmid; a phagemid; a cosmid: an artificial chromosome, for example a yeast artificial chromosome (YAC), a bacterial artificial chromosome (BAC) or a P1-derived artificial chromosome (PAC); a phage for example a λ or M13 phage, and an animal virus, etc. An animal virus that can be used as a vector includes, but is not limited to, a retrovirus (including a lentivirus), an adenovirus, an adeno-associated virus, a herpes virus (e.g. a herpes simplex virus), a poxvirus, a baculovirus, a papillomavirus, a papovavirus (e.g. SV40). In some implementations, the vector of the present application contains regulatory elements commonly used in genetic engineering, e.g., an enhancer, a promoter, an internal ribosome entry site (IRES) and other expression control elements (e.g., a transcription termination signal, or a polyadenylation signal and a poly U sequence, etc.).

In a fifth aspect, implementations of the present application further provide a host cell containing the nucleic acid described above or transformed with the vector described above.

The term "host cell" refers to a cell that can be used for introducing a vector, including but not limited to a prokaryotic cell such as Escherichia coli or Bacillus subtilis, etc.; a fungal cell such as a yeast cell or Aspergillus, etc.; an insect cell such as an S2 Drosophila cell or Sf9, etc.; or an animal cell such as a fibroblast, a CHO cell, a COS cell, a NSO cell, a HeLa cell, a BHK cell, a HEK 293 cell or a human cell, etc. The host cell is preferably a eukaryotic cell, and more preferably a mammalian cell.

In a sixth aspect, implementations of the present application provide a method for preparing an immunoglobulin, which includes expressing the host cell described above.

The implementations of the present application further provide a method for preparing an immunoglobulin, which includes subjecting a first polypeptide and a second polypeptide to fusion expression, wherein,
the first polypeptide includes an IgG variable region located at the N terminal; and
the second polypeptide includes an IgA CH2-CH3 region or an IgM CH3-CH4 region located at the C terminal.

In some implementations, for the immunoglobulin, the IgG in the first polypeptide further has a CH1 region.

In some implementations, the IgA or the IgM in the second polypeptide further has a CH1 region.

In some implementations, the first polypeptide further includes a hinge region of the IgG.

In some implementations, the second polypeptide further includes a hinge region of the IgA or the IgM.

In some implementations, the IgG in the first polypeptide further has a CH1-CH2 region.

In some implementations, the IgG in the first polypeptide further has a CH1-CH2-CH3 region.

In some implementations, the IgM in the second polypeptide further has a CH1-CH2 region.

In some implementations, the IgG is IgG1, IgG2, IgG3 or IgG4.

In some implementations, at least one of the first polypeptide and the second polypeptide includes a CH1 region.

In some implementations, at least one of the first polypeptide and the second polypeptide includes a hinge region.

The implementations of the present application further provide an immunoglobulin prepared by the aforementioned method for preparing an immunoglobulin.

In a seventh aspect, the implementations of the present application provide a method for preparing a multimer, which includes polymerizing an immunoglobulin prepared by the aforementioned method for preparing an immunoglobulin as a monomer.

In some implementations, 2, 3 or 4 immunoglobulin monomers having IgA CH2-CH3 regions are polymerized.

In some implementations, 5 immunoglobulin monomers having IgM CH3-CH4 regions are polymerized.

The implementations of the present application further provide a multimer prepared by the aforementioned method for preparing a multimer.

In an eighth aspect, the implementations of the present application provide a solid support, the surface of which is coated with the multimer described above.

In some implementations, the solid support is a plastic support, a microparticle or a membrane support; the plastic support may be a polystyrene support; the microparticle may be a magnetic microparticle; and the membrane support may be a nitrocellulose membrane, a glass cellulose membrane or a nylon membrane.

In some implementations, the solid support is selected from a test tube, an EP tube, a multiwell plate, a chromatographic column, and wells of a microtiter plate.

In the present application, the term "microparticle" can be a sphere, a near sphere, a cube, a polyhedron or an irregular shape. The diameter of the microparticle can be 10 nm-1 mm, e.g., 100 nm, 500 nm, 1 µm, 10 µm, 100 µm and 500 µm; and preferably 400 nm-10 µm.

The microparticle is preferably a magnetic microparticle, and its components contain a magnetic substance. The magnetic substance can be metal (metal elementary substance or alloy), non-metal, or a complex formed from metal and non-metal. The metal is for example iron, ferro-nickel aluminium metal, etc.; the non-metal is for example ferrite non-metal (preferably Fe₂O₃ or Fe₃O₄ magnetic nanoparticles); and the complex formed from the metal and the non-metal is for example a neodymium iron boron rubber-magnetic composite material.

The multiwell plate is preferably an enzyme label plate, which can have 8, 16, 32, 48, 64, 96 wells or more.

In a ninth aspect, implementations of the present application provide a kit or test strip containing the multimer described above or the solid support described above.

In some implementations, the test strip includes a sample pad, a binding pad, a reaction membrane and an absorption pad, and a detection region and a quality control region are disposed on the reaction membrane;
the multimer is coated on the detection region and/or added dropwise on the binding pad.

It can be easily understood that when both the detection region and the binding pad contain the multimer, the antigen epitope bound to the IgG variable region of the multimer is different, and the domain at the second polypeptide terminal can be the same or different.

In some implementations, the kit can further contain a sample pretreatment reagent (e.g., a sample purification and enrichment reagent, a lysis buffer, etc.), a cleaning solution (e.g., water, etc.), a buffer solution (e.g., PBS or Tris, etc.), a chromogenic reagent for a signal substance (e.g., ECL or DAB for a horseradish peroxidase used as a signal substance), etc.

In a tenth aspect, implementations of the present application provide a method for detecting an antigen, including:
contacting the multimer described above or the solid support described above with the antigen to form a conjugate, and
detecting the conjugate,
wherein, the antigen is an antigen capable of specifically binding with the IgG variable region.

The conjugate can be detected by any method well known to those skilled in the art, for example, a double antibody sandwich method. That is, after the antigen is contacted with the solid support, the conjugate is detected with another additional antibody (which is usually labeled with a signal substance or detected with a secondary antibody labeled with a signal substance) against the antigen.

Alternatively, a conventional antibody is used as an antibody coated on the solid support, and it is incubated with the antigen to be detected, and is detected with the multimer as a free detection antibody (which is usually labeled with a signal substance or detected with a secondary antibody labeled with a signal substance).

Alternatively, the solid support coated with the multimer cooperates with another multimer to detect the antigen, and at this time the multimer coated on the solid support and the other multimer are paired antibodies.

The aforementioned signal substance can be any one of a fluorescent substance, quantum dot, digoxin labeled probe, biotin, radioisotope, radioactive contrast agent, paramagnetic ion fluorescent microsphere, electron dense substance, chemiluminescence marker, ultrasonic contrast agent, photosensitizer, colloidal gold or enzyme. In some implementations, the signal substance is the colloidal gold, fluorescein, fluorescent microsphere, acridine ester, horseradish peroxidase, alkaline phosphatase or β-galactosidase.

In an eleven aspect, the implementations of the present application provide use of the multimer, solid support, and kit or test strip described above in immunoassay.

The implementations of the present application further provide the multimer, solid support, and kit or test strip described above for use in immunoassay.

The implementations of the present application further provide a method for conducting immunoassay, which includes conducting immunoassay with the multimer, solid support, and kit or test strip described above.

In a still further aspect, the implementations of the present application provide an immunodiagnostic kit, which includes a multimer of an IgG-IgM or IgG-IgA chimeric immunoglobulin.

In some implementations, the immunoglobulin is obtained by fusing a first polypeptide and a second polypeptide, wherein
the first polypeptide includes an IgG variable region located at the N terminal; and
the second polypeptide includes an IgA CH2-CH3 region or an IgM CH3-CH4 region located at the C terminal.

In some implementations, for the immunoglobulin, the IgG in the first polypeptide further has a CH1 region.

In some implementations, the IgA or the IgM in the second polypeptide further has a CH1 region.

In some implementations, the first polypeptide further includes a hinge region of the IgG.

In some implementations, the second polypeptide further includes a hinge region of the IgA or the IgM.

In some implementations, the IgG in the first polypeptide further has a CH1-CH2 region.

In some implementations, the IgG in the first polypeptide further has a CH1-CH2-CH3 region.

In some implementations, the IgM or IgA in the second polypeptide further has a CH1-CH2 region.

In some implementations, the IgG is IgG1, IgG2, IgG3 or IgG4.

In some implementations, at least one of the first polypeptide and the second polypeptide includes a CH1 region.

In some implementations, at least one of the first polypeptide and the second polypeptide includes a hinge region.

In some implementations, the multimer is obtained by polymerizing 2, 3 or 4 immunoglobulin monomers having IgA CH2-CH3 regions; or by polymerizing 5 immunoglobulin monomers having IgM CH3-CH4 regions.

In some implementations, the multimer is further conjugated with a signal substance.

In some implementations, the kit further includes a sample pretreatment reagent, a cleaning solution, a buffer solution and a chromogenic reagent.

In some implementations, the kit further includes a sample pad, a binding pad, a reaction membrane and an absorption pad, and a detection region and a quality control region are disposed on the reaction membrane.

In some implementations, the kit may further include another antibody, which may be a multimer or monomer.

In some implementations, the multimer can be immobilized on a solid support, and the solid support includes a nitrocellulose membrane, latex, magnetic bead or ELISA plate.

In some implementations, the multimer can be labeled with signal substances such as nanoparticles, chemiluminescent substances, fluorescent substances, radioactive substances, colloids, enzymes and the like.

The embodiments of the present application will be described in detail in connection with examples hereafter.

### Examples

In the following examples, a restriction endonuclease and a Prime Star DNA polymerase are purchased from Takara. A PMD-18T vector is purchased from Takara; a plasmid extraction kit is purchased from Tiangen; and primer synthesis and gene sequencing are completed by Invitrogen.

### Example 1 Preparation of Polynucleotides Encoding IgM and IgA Heavy Chain Constant Regions

In the present application, a polynucleotide encoding a CH region of a murine antibody could be acquired from a DNA of a murine peripheral blood cell by PCR, or from a hybridoma cell strain secreting a corresponding type of monoclonal antibody by PCR.

### Acquisition of polynucleotide encoding IgM CH region

A polynucleotide encoding a murine IgM-CH region was acquired from a genome of a hybridoma cell strain 6M9 (available from Fapon Biotech) secreting an IgM-type murine monoclonal antibody by PCR. The primers used were as follows:
forward primer sequence (5'→3'): GAG AGT CAG TCC TTC CCA AAT GTC TTC CCC CTC GTC TC (SEQ ID NO: 1); and
reverse primer sequence (5'→3'): CCC GAA TTC TTATCAATA GCA GGT GCC ACC TGT GTC AGA CAT GAT CAG (SEQ ID NO: 2).

An EcoRI restriction site (GAATTC) was introduced immediately after a stop codon TGA TAA (anticodon TTATTCA) by the reverse primer. A double stop codon was used here. A polynucleotide of about 1.4 kb was obtained by PCR technology and introduced into a pMD-18T vector for sequencing confirmation.

The polynucleotide encodes the murine IgM-CH region, and its amino acid sequence from the N terminal to the C terminal was shown as follows:

ESQSFPNVFPLVSCESPLSDKNLVAMGCLARDFLPSTISFTWNYQNNTEVIQGIRTFPTLR
TGGKYLATSQVLLSPKSILEGSDEYLVCKIHYGGKNRDLHVPIPAVAEMNPNVNVFVPPRDGF
SGPAPRKSKLICEATNFTPKPITVSWLKDGKLVESGFTTDPVTIENKGSTPQTYKVISTLTISEID
WLNLNVYTCRVDHRGLTFLKNVSSTCAASPSTDILTFTIPPSFADIFLSKSANLTCLVSNLATYE
TLNISWASQSGEPLETKIKIMESHPNGTFSAKGVASVCVEDWNNRKEFVCTVTHRDLPSPQK
KFISKPNEVHKHPPAVYLLPPAREQLNLRESATVTCLVKGFSPADISVQWLQRGQLLPQEKYV
TSAPMPEPGAPGFYFTHSILTVTEEEWNSGETYTCVVGHEALPHLVTERTVDKSTGKPTLYN
VSLIMSDTGGTCY（SEQ ID NO: 3）
.

### Acquisition of polynucleotide encoding IgA CH region

A polynucleotide encoding an IgA CH region was obtained from an isolated DNA of a murine peripheral blood cell (available from Fapon Biotech) by PCR. The primers used were as follows:
forward primer sequence (5'→3'): GAG TCT GCG AGA AAT CCC A CC ATC TAC CCA CTG ACA CTC (SEQ ID NO: 4); and
reverse primer sequence (5'→3'): CCC GAA TTC TCA GTA GCA GAT GCC ATC TCC CTC TGA CAT GAT (SEQ ID NO: 5).

An EcoRI restriction site was introduced immediately after a stop codon TGA (anticodon TCA) by the reverse primer. A polynucleotide of about 1 kb was obtained by PCR technology and introduced into a pMD-18T vector for sequencing confirmation.

The polynucleotide encodes the murine IgA-CH region, and its amino acid sequence from the N terminal to the C terminal was shown as follows:

ESARNPTLYPLTLPPALSSDPVIIGCLIHDYFPSGTMNVTWGKSGKDITTVNFPPALASGG
RYTMSSQLTLPAVECPEGESVKCSVQHDSNPVQELDVNCSGPTPPPPITIPSCQPSLSLQRPALE
DLLLGSDASITCTLNGLRNPEGAVFTWEPSTGKDAVQKKAVQNSCGCYSVSSVLPGCAERW
NSGASFKCTVTHPESGTLTGTIAKVTVNTFPPQVHLLPPPSEELALNELLSLTCLVRAFNPKEV
LVRWLHGNEELSPESYLVFEPLKEPGEGATTYLVTSVLRVSAETWKQGDQYSCMVGHEALP
MNFTQKTIDRLSGKPTNVSVSVIMSEGDGICY（SEQ ID NO: 6）.

The polynucleotides encoding the CH1, CH2, CH3 and CH4 domains of the IgM CH region or a combination thereof, or encoding the CH1, CH2 and CH3 domains of the IgA CH region or a combination thereof, were prepared in a similar manner.

### Example 2 Construction of Expression Plasmid of IgG/IgM Heterozygous recombinant Antibody

In this example, an anti-Pan-PLDH 9G7 monoclonal antibody (hereinafter referred to as MA 9G7) was employed as an IgG antibody, and the sequence of this monoclonal antibody was stated in Chinese Patent Publication No. CN111363044A, which was incorporated herein by reference. The antibody could be selected from any antibody having any group of mutations from the mutation combinations 1-56 in paragraphs [0087]-[0089] in CN111363044A, e.g., a mutation combination 32, which had similar properties.

The sequence of the IgM itself has the characteristics of a multimer. Firstly, the polynucleotide encoding a segment 1 of a heavy chain variable region (VH) of the MA 9G7 was linked with the polynucleotide encoding the IgM CH region by bridge PCR. The amino acid sequence of the segment 1 of the VH region of the MA 9G7 was as follows: 
QVQLQQPGAELVRPGASVKLSCKASGYSFTNYWMNWVKQRPGQGLEWIGMIHPSDSE
TRLNQKFKDKATLTVDKSSSTAYMQLSSLTAEDSAVYYCARSGDFYTYYFDYWGQGTTLTV
SS（SEQ ID NO: 7）
 a polynucleotide of about 1.8 kb was obtained by PCR, and was ligated into a pcDNA^{™} 3.4 TOPO^{®} vector cleaved with a corresponding restriction enzyme, by Hindlll/EcoRl double enzyme digestion. The obtained vector was named pcDNA3.4A-9G7TB1VCH.

Then, a polynucleotide encoding a segment 2 of the VH region of the MA 9G7 was selected and linked with a polynucleotide encoding the starting from the CH2 to the C terminal of the IgM CH region by bridge PCR. The amino acid sequence of the segment 2 of the VH region of the MA 9G7 was as follows: 
QVQLQQPGAELVRPGASVKLSCKASGYSFTNYWMNWVKQRPGQGLEWIGMIHPSDSE
TRLNQKFKDKATLTVDKSSSTAYMQLSSLTAEDSAVYYCARSGDFYTYYFDYWGQGTTLTV
SSAKTTPPSVYPLAPGCGDTTGSSVTLGCLVKGYFPESVTVTWNSGSLSSSVHTFPALLQSGL
YTMSSSVTVPSSTWPSQTVTCSVAHPASSTTVDKKLEPSGPIST（SEQ ID NO: 8）
 a DNA fragment of about 1.8 KB was obtained by PCR, and was ligated into the pcDNA^{™} 3.4 TOPO^{®} vector which was abbreviated as pcDNA3.4A-9G7TB2VCH, by Hindlll/EcoRl double enzyme digestion.

Because the polymerization characteristics of the IgM depended on the heavy chain, the light chain clone of the antibody in this example was constructed with the construction method of a light chain plasmid disclosed in Chinese Patent Publication No. CN111363044A.

### Example 3 Preparation for Expression of MA-9G7TB1 and MA-9G7TB2 IgG/IgM Heterozygous recombinant Antibodies

### 1. Linearization of recombinant antibody expression plasmid

The following enzyme digestion system was prepared: 50 ul of a restriction enzyme Buffer, 100 ug/tube of a recombinant plasmid, 10 ul of a Pvu I enzyme, and sterile water which was supplemented to 500 ul. Enzyme digestion was conducted with the enzyme digestion system in water bath at 37°C overnight. Firstly, it was extracted sequentially with phenol/chloroform/isoamylol (25:24:1) (lower layer) with the same volume as the enzyme digestion system followed by chloroform (water phase); allowed to stand on ice with 0.1 times the volume (water phase) of 3M sodium acetate and 2 times the volume of ethanol to precipitate the DNA. The DNA precipitate was rinsed with 70% ethanol to remove the organic solvent, redissolved with appropriate amount of sterilized water after the ethanol volatilized completely, and finally determined for the concentration.

### 2. Stable transfection of recombinant antibody expression plasmid, and pressure screening of stable cell strain

The pcDNA3.4A-9G7TB1VCH plasmid and a light-chain plasmid were diluted to 40 ug/100 ul with ultrapure water in a ratio of 1:1. A new centrifuge tube was taken and added with CHO cells of which the concentration was adjusted to 1.43×10⁷ cells/ml. 100 ul of the plasmids were mixed with 700 ul of the cells and transferred into an electroporation cup for electrotransfection. The cells were cultured continually after the transfection and counted on the next day. The cells were subjected to 25 umol/L MSX 96 well pressure culture for about 25 days.

A clone well with grown cells was observed and marked under a microscope, and the confluence was recorded. The culture supernatant was taken and sent for sample detection. Cell strains with high antibody concentration and relative concentration were selected and transferred into a 24-well plate, and transferred to a 6-well plate after about 3 days. After 3 days, breed conservation was performed and batch culture was conducted, wherein the cells were adjusted to a density of 0.5×10⁶ cells/ml and subjected to batch culture at a volume of 2.2 ml, and the breed conservation was performed with a cell density of 0.3×10⁶ cells/ml at 2 ml. 7 days later, the supernatant of the batch culture in the 6-well plate was sent for sample detection, and cell strains with relatively small antibody concentration and cell diameter were selected and transferred into a TPP tube for breed conservation and passage, so as to obtain cell strains expressing the MA-9G7TB1 IgG/IgM heterozygous recombinant antibody.

The pcDNA3.4A-9G7TB2VCH plasmid and the light-chain plasmid were transfected and screened as described above for the pcDNA3.4A-9G7TB1VCH plasmid, so as to obtain a cell strain expressing a MA-9G7TB2 IgG/IgM heterozygous recombinant antibody.

### 3. Cell expansion culture

After thawed, the cells were firstly inoculated in a shake flask with a specification of 125 ml containing a 100% Dynamis medium at an inoculation volume of 30 ml, and placed into a shaker with a rotating speed of 120 r/min and a temperature of 37°C and 8% carbon dioxide for culture. After 72 h of culture, the cells were inoculated at the inoculation density of 5×10⁵ cells/ml for expansion culture at an expanded culture volume calculated according to production requirements, and the used medium was a 100% Dynamis medium. Thereafter, expansion culture was conducted every 72 h. When the cell quantity met the production requirements, production was conducted with an inoculation density strictly controlled at about 5×10⁵ cells/ml.

### 4. Shake flask production and purification

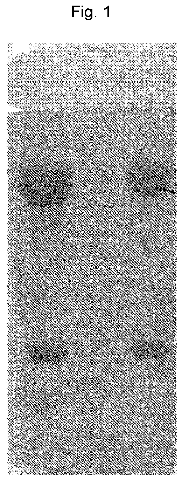

Shake flask production parameters: a rotation speed of a shaker at 120 r/min, a temperature of 37°C, and a carbon dioxide content of 8%. Fed-batch feeding: upon 72 h of culture in a shake flask, daily feeding was started, with HyClone^{™} Cell Boost^{™} Feed 7a being fed in a fed-batch manner daily at amount of 3% of an initial culture volume and HyClone^{™} Cell Boost^{™} Feed 7b being fed in a fed-batch manner daily at amount of one thousandth of the initial culture volume, until day 12 (feeding at day 12). 3 g/L of glucose was supplemented on the sixth day. The sample was collected on day 13. A multimeric antibody of MA-9G7TB1 and MA-9G7TB2 IgG/IgM heterozygous recombinant antibodies was obtained in two steps by affinity chromatography utilizing two fillers of captoL (cytiva) and CHT (Bio-Rad). 10 ul of the purified antibody (with a concentration of 1 mg/ml) was taken for reducing SDS-PAGE, and the electrophoresis result was as shown in Fig. 1. In Fig. 1, the left lane was a band of the MA-9G7TB1 IgG/IgM recombinant antibody, and the right lane was a band of the MA-9G7TB2 IgG/IgM recombinant antibody. Two bands were shown on each lane. The relative molecular mass (Mr) of one band was 70-75 kD (heavy chain) and the Mr of the other band was 20-35 kD (light chain).

### Example 4 Application of MA-9G7TB1 and MA-9G7TB2 IgG/IgM Heterozygous recombinant Antibodies

### Preparation of MA-9G7TB1 Colloidal Gold Detection Test Strip

### 1. Preparation of nitrocellulose membrane

Preparation of nitrocellulose membrane: the MA-9G7TB1 recombinant antibody was diluted to 1-5 mg/ml with a coating buffer, and streaked with a T line close to the colloidal gold end as a detection line. A goat anti-murine IgG antibody (produced by Fapon Biotech Co., Ltd., Cat. No. BA-PAB-MU0001) was diluted to 1-5 mg/ml with the coating buffer, and streaked with a C line close to the absorption pad as a control line. The nitrocellulose membrane was dried at 37°C and encapsulated for later use.

### 2. Preparation of colloidal gold and colloidal gold-labeled monoclonal antibody

### 2.1 Preparation of colloidal gold

1% chloroauric acid was diluted to 0.01% with double distilled deionized water, put in an electric furnace for boiling, added with 2 ml of 1% trisodium citrate per 100 ml of the 0.01% chloroauric acid, and boiling continually. The heating was stopped until the liquid was bright red, and the water loss was made up after the solution was cooled to room temperature. The prepared colloidal gold had a pure and transparent appearance, had no precipitation and floating objects, and had a validity period of one week.

### 2.2 Preparation of colloidal gold-labeled antibody

The colloidal gold was adjusted to a pH value of 8.2 with 0.1M potassium carbonate, added with another MA-labeled antibody (available from Fapon Biotech) according to 8-10 µg antibody/ml colloidal gold, mixed evenly with a magnetic stirrer for 30 min, added with BSA under stirring until the final concentration was 1%, and allowed to stand for 1 h. The liquid was centrifuged at 13,000 rpm and 4°C for 30 min. The supernatant was discarded. The precipitate was washed twice with a labeling and washing preservation solution, resuspended with the labeling and washing preservation solution at one tenth of the volume of the initial colloidal gold, and placed at 4°C for later use, with a validity period of one week.

### 3. Preparation of gold label pad

A gold label pad was soaked in a blocking solution for 30 min, and then oven dried at 37°C. Then, the prepared colloidal gold-labeled antibody was evenly spread on the gold label pad with 20 square centimeters being spread per milliliter of the solution, freeze-dried, encapsulated and placed at 4°C for later use.

### 4. Preparation of test strip sample pad

The sample pad was soaked in a blocking solution (containing BSA) for 30 min, then oven dried at 37°C, encapsulated and placed at 4°C for later use.

### 5. Assembly of detection test strip

An absorbent pad (purchased from Millipore), the nitrocellulose membrane, the gold label pad and the sample pad were disposed on a non-absorbent support sheet and cut into small strips with a width of 3 mm. Every ten strips were packaged as a packet, added with a drying agent, and encapsulated in vacuum to obtain the MA-9G7TB1 colloidal gold test strip.

The colloidal gold detection test strips were respectively prepared using the MA-9G7TB2 recombinant antibody and the MA-9G7 recombinant antibody according to the method described for preparing the MA-9G7TB1 colloidal gold test strip, and the mass concentration of the T-line heterozygous recombinant antibody was the same as that of the control antibody.

### 6. Application of colloidal gold detection test strip

The assembled test strip was utilized to detect whether the material to be tested contained a Pan-PLDH protein (hereinafter referred to as a MA protein), so as to determine the role of the MA antibody in the detection of the MA protein. During detection, the MA protein first bound with the colloidal gold-labeled MA antibody to form a MA-colloidal gold-labeled MA antibody complex. Due to capillary action, the MA-colloidal gold-labeled MA antibody complex swam forward along the nitrocellulose membrane. When it reached the detection line, the MA-colloidal gold-labeled MA antibody complex would bind with the streaked MA-coated antibody to form a MA antibody -MA-colloidal gold-labeled MA antibody complex, which would be enriched on the detection line to form a red precipitation line. Table 1 showed the corresponding detection data. The depth of the red precipitation line indicated the strength of the reaction. The reactivity was stronger when the red color was deeper, and otherwise weaker. Meanwhile, the strength of the reaction was represented by a combination of a letter C and a number. The reactivity was stronger when the number after C was smaller, and otherwise weaker. No formation of the red precipitation line was represented with B.

**Table 1**

| Coating | | MA-9G7 | MA- 9G7TB1 | MA- 9G7TB2 |
|---|---|---|---|---|
| MA quality control product | PK2-MA1 (100 ng/ml) | C4 | C3 | C3 |
| | PK2-MA8 (100 ng/ml) | C9 | C8 | C7 |
| | Commercially-available PV-PFDH (100 ng/ml) | C3 | C3 | C3+ |
| | Commercially-available PF-PLDH (100 ng/ml) | C5 | C3 | C3 |
| Positive specimen | PV positive blood | C4 | C4 | C4+ |
| | PV positive blood | C4 | C3 | C3 |
| Comment: "+" represented the degree of reaction being 0.5 color cards stronger; | | | | |

Example 5 Construction, Preparation and Application in Colloidal Gold Platform of SARS-CoV-2 3E50TB2 and 5F27TB2 IgG/IgM Heterozygous recombinant Antibodies

In this example, a 3E50 antibody was used as the IgG antibody, which was stated in Chinese Patent Publication No. CN112239501A, which was incorporated herein by reference. The antibody could be selected from any antibody having any group of mutations from the mutation combinations 1-68 in paragraphs [0049]-[0052] in CN112239501A, e.g., a mutation combination 50, which had similar properties. In this example, a 5F27 antibody was used as the IgG antibody, which was stated in Chinese patent CN112239500A, which was incorporated herein by reference. The antibody could be selected from any antibody having any group of mutations from the mutation combinations 1-42 in paragraphs [0048]-[0049] in CN112239500A, e.g., a mutation combination 20, which had similar properties.
1. The expression plasmids of the SARS-CoV-2 3E50TB2 IgG/IgM and 5F27TB2 IgG/IgM recombinant antibodies were constructed according to the construction manner of the MA-9G7TB2 expression plasmid in Example 2.
The expressed SARS-CoV-2 3E50TB2 and 5F27TB2 IgG/IgM recombinant antibodies would be antibodies obtained by splicing respective VH + CH1 segments (including hinge regions) of the 3E50 and 5F27 IgGs with the CH2-CH3-CH4 segment of the IgM mentioned above.
2. The SARS-CoV-2 3E50TB2 and 5F27TB2 IgG/IgM recombinant antibodies were prepared according to the manner of expressing the MA-9G7TB2 recombinant antibody in Example 3.

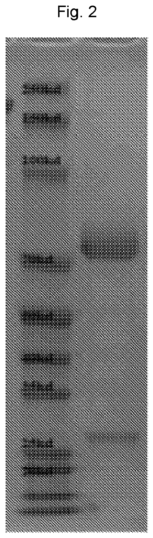

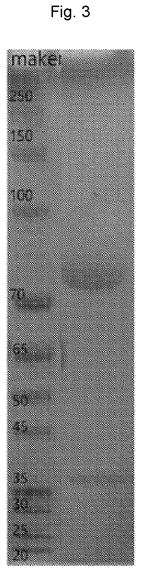

Figs. 2 and 3 respectively showed the electrophoresis results of the 3E50TB2 and 5F27TB2 IgG/IgM heterozygous recombinant antibodies.
3. The application of the SARS-CoV-2 3E50TB2 and 5F27TB2 IgG/IgM recombinant antibodies was evaluated according to the method of Example 4, and the specific performance evaluation results were as shown in the table below:

**Table 2 Detection data of 3E50 and 3E50TB2 respectively used as colloidal gold coatings cooperated with the 5F27 label**

| Coating | | 3E50-coated | 3E50TB2-coated |
|---|---|---|---|
| Labeling | | 5F27-labelled | |
| Antigen | 25 ng/ml | C4+ | C3+ |
| | 2 ng/ml | C7+ | C5 |
| | 500 pg/ml | C9+ | C7 |
| | 100 pg/ml | B+ | C9+ |
| | 25 pg/ml | B | B+ |
| Vaccine | 1:5K | C8 | C7+ |
| | 1:30K | B | B+ |
| | 1:90K | B | B+ |

| | | | |
|---|---|---|---|
| Note: the vaccine referred to an inactivated novel coronavirus sample. | | | |

Table 3 Detection data of 5F27 and 5F27TB2 respectively used as colloidal gold coatings cooperated with the 3E50 label

| Coating | | 5F27-coated | 5F27TB2-coated |
|---|---|---|---|
| Labeling | | 3E50-labelled | |
| Antigen | 25 ng/ml | C3+ | C2+ |
| | 2 ng/ml | C5+ | C4+ |
| | 500 pg/ml | C7+ | C6+ |
| | 100 pg/ml | C9+ | C8 |
| | 25 pg/ml | B | B+ |
| Vaccine | 1:5K | C6+ | C6+ |
| | 1:30K | C8 | C8 |
| | 1:90K | C9 | C9++ |

Table 4 Test results of 3E50TB2 and 5F27TB2 respectively used as coatings cooperated with labels

| Coating | | 5F27-coated | 5F27TB2-coated | 3E50-coated | 3E50TB2-coated | 5F27TB2-coated | 5F27TB2-labelled |
|---|---|---|---|---|---|---|---|
| Labeling | | 3E50-labelled | | 5F27-labelled | | 3E50TB2-labelled | 3E50TB2-coated |
| Antige n | 25 ng/ml | C3+ | C2+ | C4+ | C3+ | C1+ | C1+ |
| | 2 ng/ml | C5+ | C4+ | C7+ | C5 | C3 | C3 |
| | 500 pg/ml | C7+ | C6+ | C9+ | C7 | C5++ | C5+ |
| | 100 pg/ml | C9+ | C8 | B+ | C9+ | C7+ | C7+ |
| | 25 pg/ml | B | B+ | B | B+ | C9+ | C9+ |
| Vacci ne | 1:5K | C6+ | C6+ | C8 | C7+ | C4+ | C4+ |
| | 1:30K | C8 | C8 | B | B+ | C7++ | C7+ |
| | 1:90K | C9 | C9++ | B | B+ | C9+ | C9+ |

### Example 6 Construction, Preparation and Application in Fluorescent Chromatography Platform of CTNI 21C5TB2 IgG/IgM Heterozygous recombinant Antibody

In this example, an Anti-cTnl-21C5 monoclonal antibody (hereinafter referred to as CTNI 21C5) was employed as an IgG antibody, and the sequence of this monoclonal antibody was stated in Chinese Patent Publication No. CN111018983A, which was incorporated herein by reference. The antibody could be selected from any antibody having any group of mutations from the mutation combinations 1-56 in paragraphs [0080]-[0084] in CN111018983A, e.g., a mutation combination 37, which had similar properties.
1. The expression plasmid of the CTNI 21C5TB2 IgG/IgM recombinant antibody was constructed according to the construction manner of the MA-9G7TB2 expression plasmid in Example 2.

The expressed CTNI 21C5TB2 IgG/IgM recombinant antibody would be an antibody obtained by splicing VH + CH1 segments (including a hinge region) of the CTNI 21C5 IgG with the CH2-CH3-CH4 segment of the IgM mentioned above.

The expression plasmid of the CTNI 21C5TB1 IgG/IgM recombinant antibody was constructed according to the construction manner of the MA-9G7TB1 expression plasmid in Example 2.

The expressed CTNI 21C5TB1 IgG/IgM recombinant antibody would be an antibody obtained by splicing the heavy chain variable region (VH) segment of the CTNI 21C5 IgG with the CH segment of the IgM mentioned above.

Additionally, the expression plasmids of CTNI 21C5TB3 and CTNI-TBQ IgG/IgM recombinant antibodies were constructed. Specifically, the expressed CTNI-TBQ IgG/IgM recombinant antibody would be an antibody obtained by splicing the CTNI 21C5 full-length antibody (including an entire constant region) with the CH3-CH4 segment of the IgM. Specifically, the expressed CTNI 21C5TB3 IgG/IgM recombinant antibody would be an antibody obtained by splicing the VH + CH1-CH2 segment (including a hinge region) of the IgG with the CH3-CH4 segment of the IgM.

2. The CTNI 21C5TB2, CTNI 21C5TB3, full-length antibody CTNI-TBQ and CTNI 21C5TB1 IgG/IgM recombinant antibody were prepared according to the preparation manner of expressing the MA-9G7TB2 recombinant antibody in Example 3.

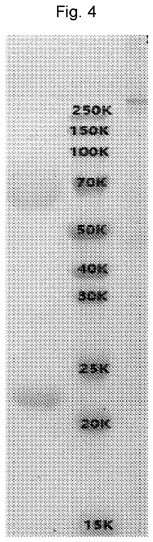

Fig. 4 showed the electrophoresis result of the 21C5TB2 heterozygous recombinant antibody.

3. Application of CTNI 21C5TB2 recombinant antibody in fluorescent chromatography platform:
3.1 Preparation of fluorescent chromatography detection reagent cards for CTNI 21C5TB2, CTNI 21C5TB3, full-length antibody CTNI-TBQ, CTNI 21C5TB1 and CTNI 21C5
3.1.1 Preparation of nitrocellulose membrane

Preparation of nitrocellulose membrane: the CTNI 21C5TB2 recombinant antibody was diluted to 1-2 mg/ml with a coating buffer, and streaked with a T line (detection line) being close to the gold label pad end and about 5 mm away from the gold label pad end. A goat anti-murine IgG antibody (produced by Fapon Biotech Co., Ltd., Cat. No. BA-PAB-MU0001) was diluted to 1-2 mg/ml with the coating buffer, and streaked with a C line (control line) being close to the absorption pad and about 3 mm away from the absorption pad. The nitrocellulose membrane was dried at 37°C and encapsulated for later use.

### 3.1.2 Preparation of Monoclonal Antibody Labeled by Fluorescent Chromatography

1 ml of fluorescent microspheres with a solid content of 1% were taken, centrifuged to remove the supernatant, then added with a label activating solution having the same volume as the microspheres and mixed evenly by ultrasound, then added with activating agents EDC (with a final concentration of 5 mg/ml) and NHS (with a final concentration of 5 mg/ml), mixed evenly by shaking with protection from light for 20 min, and then centrifuged to remove the supernatant. The precipitate was added with a label coupling solution having the same volume as the microspheres and mixed evenly by ultrasound, then added with 0.2-1 mg of another CTNI-labeled antibody, mixed evenly by shaking with protection from light for 3 h, finally added with a label blocking solution for blocking, and mixed by shaking with protection from light for 45 min, and then the labeling was terminated. The solution was centrifuged to remove the supernatant. The microspheres were redissolved with a microsphere label preservation solution, mixed evenly by ultrasound, and placed at 4°C for later use, with a validity period of 1 month.

### 3.1.3 Preparation of Fluorescent-Labeled Pad

After a marker was diluted by 5-10 times with a marker diluent, the diluted marker was sprayed onto a glass fiber pad with a liquid spraying instrument. The labelled pad was encapsulated and placed at 4°C for later use.

### 3.1.4 Preparation of test strip sample pad

The glass fiber pad was soaked in a blocking solution (containing BSA) for 30 min, and then oven dried at 37°C, and the sample pad was encapsulated and placed at 4°C for later use.

### 3.1.5 Assembly of detection reagent card

The absorbent pad (purchased from Millipore), the nitrocellulose membrane, the labelled pad and the sample pad were disposed on a non-absorbent support sheet, cut into small strips with a width of 3 mm, packed into a card case with each card being packed as a packet, added with a drying agent, and encapsulated in vacuum in an aluminum foil bag to obtain a CTNI 21C5TB2 fluorescent chromatography detection reagent card.

Fluorescent chromatography detection reagent cards were prepared respectively with the CTNI 21C5TB3, full-length antibody CTNI-TBQ, CTNI 21C5TB1 and CTNI 21C5 fluorescent chromatography detection reagent cards according to the method described for preparing the CTNI 21C5TB2 fluorescent chromatography detection reagent card. The mass concentrations of theT-line heterozygous recombinant antibodies of the CTNI 21C5TB1, full-length antibody CTNI-TBQ, CTNI 21C5TB2, CTNI 21C5TB3 IgG/IgM heterozygous recombinant antibodies were the same as those of the control antibodies.

### 3.2 Test results of CTNI 21C5TB2 recombinant antibody in fluorescent chromatography platform

A purchased CTNI antigen was used as a sample to be tested. A test card was taken out by tearing an aluminum foil bag. For each test sample, 50 µL of the sample was pipetted, added into a sample diluent and well mixed fully. Then 50 µL of the solution was pipetted and added into a sample addition well of a test card; the reaction time of the test card after the sample addition was 15 min; and a fluorescence detector would automatically conduct testing and read the results. Meanwhile, 30 clinical assignment specimens collected from a hospital were used as detection samples to detect the specimen correlation r of the 21C5TB2 or 21C5 antibody. The specific test results were as shown in Table 5, indicating that the detection sensitivity of the 21C5TB2 antibody was greater than that of the 21C5. In the same way, according to the aforementioned testing method, the measured correlations r of the 30 specimens detected with the 21C5TB1, 21C5TB3 and 21C5TBQ antibodies were all above 0.9, and the sensitivity was 21C5TB2 > 21C5TB3 > 21C5TBQ > 21C5TB1.

**Table 5**

| Clinical assignment specimen (ng/ml) | 21C5 | 21C5TB2 |
|---|---|---|
| 50 | 2.168 | 6.209 |
| 18 | 0.746 | 3.022 |
| 10.5 | 0.450 | 1.700 |
| 3 | 0.180 | 0.460 |
| 0.7 | 0.045 | 0.110 |
| 0.15 | 0.026 | 0.040 |
| 0.08 | 0.020 | 0.027 |
| 0.04 | 0.017 | 0.023 |
| 0 | 0.015 | 0.015 |
| Detecting the correlation r of 30 specimens | 0.96 | 0.98 |

### Example 7 Construction, Preparation and Application in Fluorescent Chromatography Platform of CKMB 10C5TB4lgG/lgA Heterozygous recombinant Antibody

In this example, an Anti-CKMB 10C5 monoclonal antibody (hereinafter referred to as CKMB 10C5) was employed as an IgG antibody, and the sequence of this monoclonal antibody was stated in Chinese Patent Publication No. CN111349168A, which was incorporated herein by reference. The antibody could be randomly selected from any antibody having any group of mutations from the mutation combinations 1-56 in paragraphs [0096]-[0098] in CN111349168A, e.g., a mutation combination 11, which had similar properties.

### 1. Construction of expression plasmid of CKMB 10C5TB4 IgG/lgA heterozygous recombinant antibody

Firstly, the polynucleotide encoding the VH region + partial CH1 segment of the CKMB 10C5 was linked with the polynucleotide encoding a fragment starting from the hinge region to the C terminal of the IgA CH region by bridge PCR. a DNA fragment of about 1.5 kb was obtained by PCR, and was ligated into the pcDNA^{™} 3.4 TOPO^{®} vector which was abbreviated as pcDNA3.4A-10C5TB3VCH, by Hindlll/EcoRl double enzyme digestion.

The expression plasmid of the CKMB 10C5TB1 IgG/IgM recombinant antibody was constructed according to the construction manner of the MA-9G7TB1 expression plasmid in Example 2.

The expressed CKMB 10C5TB1 IgG/IgM recombinant antibody would be an antibody obtained by splicing the VH segment of the CKMB 10C5lgG with the CH segment of the IgM mentioned above.

Additionally, the expression plasmid of the CTNI-TBQ IgG/IgM recombinant antibody was constructed. Specifically, the expressed CTNI-TBQ IgG/IgM recombinant antibody would be an antibody obtained by splicing the CKMB 10C5 full-length antibody (including an entire constant region) with the same fragment of the IgM mentioned above.

The light chain clone of the antibody in this example was constructed with the construction method of a light chain plasmid disclosed in Chinese Patent Publication No. CN111349168A.

2. The CKMB 10C5TB4, CKMB 10C5TB1 and CTNI-TBQ IgG/IgM recombinant antibodies were prepared according to the manner for expressing the MA-9G7TB2 recombinant antibody in Example 3, and the antibodies were obtained in two steps through affinity chromatography by utilizing two fillers of captoL (cytiva) and CHT (Bio-Rad).

3. Application of CKMB 10C5TB4 recombinant antibody in fluorescent chromatography platform:
3.1 The preparation of fluorescent chromatography detection reagent cards for the full-length antibodies CKMB-TBQ, CKMB 10C5 and CKMB 10C5TB4 was the same as that described in Example 6, except that the heterozygous recombinant antibody in each group of this example was an IgA dimer, and the mass concentrations of the T-line heterozygous recombinant antibodies of the full-length antibodies CKMB-TBQ, CKMB 10C5TB4 IgG/lgA heterozygous recombinant antibodies were the same as those of control antibodies.
3.2 Test results of CKMB 10C5TB4 recombinant antibody in fluorescent chromatography platform

A purchased CKMB antigen was used as a sample to be tested. A test card was taken out by tearing an aluminum foil bag. For each test sample, 50 µL of the sample was pipetted, added into a sample diluent and well mixed fully. Then 50 µL of the solution was pipetted and added into a sample addition well of a test card; the reaction time of the test card after the sample addition was 15 min; and a fluorescence detector would automatically conduct testing and read the results. Meanwhile, 30 clinical assignment specimens collected from a hospital were used as detection samples to detect the specimen correlation r of the 10C5TB4 or 10C5 antibody. The specific test results were as shown in Table 6, and it could be seen that the detection sensitivity of the 10C5TB4 antibody was greater than that of the 10C5, and the correlations r of the 30 specimens as detected were all above 0.9. In the same way, according to the aforementioned testing method, the measured correlations r of the 30 specimens detected with the 10C5TB1 and 10C5TBQ antibodies were all above 0.9, and the sensitivity was 10C5TB4 > 10C5TBQ > 10C5TB1.

**Table 6**

| CKMB commercially-available antigen | 10C5 | 10C5TB4 |
|---|---|---|
| ng/ml | T/C | T/C |
| 300 | 10.280 | 12.266 |
| 150 | 7.034 | 8.170 |
| 50 | 4.691 | 6.403 |
| 20 | 2.136 | 2.876 |
| 5 | 0.406 | 0.722 |
| 2 | 0.163 | 0.266 |
| 0.5 | 0.040 | 0.110 |
| 0 | 0.014 | 0.030 |
| Detecting the correlation r of 30 specimens (0-50 ng/ml) | 0.96 | 0.96 |
| Detecting the correlation r of 30 specimens (0-300 ng/ml) | 0.99 | 0.99 |

The technical features of the aforementioned examples can be arbitrarily combined. To simplify the description, we do not describe all possible combinations of the technical features in the aforementioned examples. However, as long as there is no contradiction in the combination of these technical features, it should be considered as the scope stated in this specification.

The examples described above are merely illustrative of several implementations of the present application, the description of them is more specific and detailed, but cannot be construed as limiting the scope of the present disclosure accordingly. It should be noted that, several variations and modifications can be made by those of ordinary skills in the art, under the premise of not departing from the concept of the present application, and these variations and modifications all fall within the claimed scope of the present application. Therefore, the claimed scope of the patent of the present application shall be determined by the appended claims.

## Claims

1. An immunoglobulin obtained by fusing a first polypeptide and a second polypeptide, wherein
the first polypeptide comprises an IgG variable region located at the N terminal; and
the second polypeptide comprises an IgA CH2-CH3 region or an IgM CH3-CH4 region located at the C terminal.

2. The immunoglobulin as claimed in claim 1, wherein the IgG in the first polypeptide further has a CH1 region;
optionally, the IgA or the IgM in the second polypeptide further has a CH1 region;
optionally, the first polypeptide further comprises a hinge region of the IgG;
optionally, the second polypeptide further comprises a hinge region of the IgA or the IgM;
optionally, the IgG in the first polypeptide further has a CH1-CH2 region;
optionally, the IgG in the first polypeptide further has a CH1-CH2-CH3 region;
optionally, the IgM or the IgA in the second polypeptide further has a CH1-CH2 region;
optionally, the IgG is IgG1, IgG2, IgG3 or IgG4;
preferably, at least one of the first polypeptide and the second polypeptide comprises a CH1 region; and
preferably, at least one of the first polypeptide and the second polypeptide comprises a hinge region.

3. A multimer obtained by polymerizing the immunoglobulin as claimed in claim 1 or 2 as a monomer, wherein:
optionally, the multimer is obtained by polymerizing 2, 3 or 4 immunoglobulin monomers having IgA CH2-CH3 regions; or by polymerizing 5 immunoglobulin monomers having IgM CH3-CH4 regions; and
optionally, the multimer is further conjugated with a signal substance.

4. An isolated nucleic acid encoding the immunoglobulin as claimed in claim 1 or 2.

5. A vector comprising the nucleic acid as claimed in claim 4.

6. A host cell comprising the nucleic acid as claimed in claim 4 or transformed with the vector as claimed in claim 5.

7. A method for preparing an immunoglobulin, comprising expressing the host cell as claimed in claim 6.

8. A method for preparing an immunoglobulin, comprising subjecting a first polypeptide and a second polypeptide to fusion expression, wherein:
the first polypeptide comprises an IgG variable region located at the N terminal; and
the second polypeptide comprises an IgA CH2-CH3 region or an IgM CH3-CH4 region located at the C terminal.

9. The method as claimed in claim 8, wherein the IgG in the first polypeptide further has a CH1 region;
optionally, the IgA or the IgM in the second polypeptide further has a CH1 region;
optionally, the first polypeptide further comprises a hinge region of the IgG;
optionally, the second polypeptide further comprises a hinge region of the IgA or the IgM;
optionally, the IgG in the first polypeptide further has a CH1-CH2 region;
optionally, the IgG in the first polypeptide further has a CH1-CH2-CH3 region;
optionally, the IgM or the IgA in the second polypeptide further has a CH1-CH2 region;
optionally, the IgG is IgG1, IgG2, IgG3 or IgG4;
preferably, at least one of the first polypeptide and the second polypeptide comprises a CH1 region; and
preferably, at least one of the first polypeptide and the second polypeptide comprises a hinge region.

10. An immunoglobulin prepared by the method as claimed in any one of claims 7-9.

11. A method for preparing a multimer, comprising polymerizing an immunoglobulin prepared by the method as claimed in any one of claims 7-9 as a monomer;
wherein optionally, the multimer is obtained by polymerizing 2, 3 or 4 immunoglobulin monomers having IgA CH2-CH3 regions; or by polymerizing 5 immunoglobulin monomers having IgM CH3-CH4 regions.

12. A multimer prepared by the method as claimed in claim 11.

13. A solid support of which a surface is coated with the multimer as claimed in claim 3 or 12, wherein:
optionally, the solid support is a plastic support, a microparticle or a membrane support.

14. A kit or test strip comprising the multimer as claimed in claim 3 or 12 or the solid support as claimed in claim 13, wherein:
optionally, the test strip comprises a sample pad, a binding pad, a reaction membrane and an absorption pad, and a detection region and a quality control region are disposed on the reaction membrane;
the multimer is coated on the detection region and/or added dropwise on the binding pad; and
optionally, the kit comprises a sample pretreatment reagent, a cleaning solution, a buffer solution and a chromogenic reagent.

15. A method for detecting an antigen, comprising:
contacting the multimer as claimed in claim 3 or 12 or the solid support as claimed in claim 13 with the antigen to form a conjugate, and
detecting the conjugate,
wherein, the antigen is an antigen capable of specifically binding with the IgG variable region.

16. Use of the multimer as claimed in claim 3 or 12, the solid support as claimed in claim 13 or the kit or test strip as claimed in claim 14 in immunoassay.

17. The multimer as claimed in claim 3 or 12, the solid support as claimed in claim 13 or the kit or test strip as claimed in claim 14 for use in immunoassay.

18. A method for conducting immunoassay, comprising using the multimer as claimed in claim 3 or 12, the solid support as claimed in claim 13 or the kit or test strip as claimed in claim 14 to detect an antigen.
